# EUROPEAN PATENT SPECIFICATION

(11) **EP 1 991 859 B1**
(45) Date of publication and mention of the grant of the patent: **02.08.2017**
(21) Application number: 06807092.9
(22) Date of filing: 09.10.2006
(51) Int. Cl.: G01N 21/85, G01N 21/31, G01N 33/28, G01N 21/53, G01N 21/09

(54) **PROBE, SENSOR AND MEASUREMENT METHOD**
SONDENSENSOR UND MESSVERFAHREN
SONDE, CAPTEUR ET PROCÉDÉ DE MESURE

(30) Priority: 10.10.2005 CH 16352005
(43) Date of publication of application: 19.11.2008
(73) Proprietor: FAUDI Aviation GmbH, 35260 Stadtallendorf (DE)
(72) Inventor: BUDNIK, Ralf, CH-4052 Basel (CH); DANIGEL, Harald, CH-4052 Basel (CH)
(74) Representative: Patentanwälte Olbricht Buchhold Keulertz
(86) International application number: PCT/EP2006/067206
(87) International publication number: WO 2007/042501

(56) References cited:
- WO-A-00/02034
- JP-A- 2000 266 668
- US-A- 4 497 577
- US-A- 5 007 740
- US-A1- 2003 133 112
- US-A1- 2004 141 179

## Description

The present invention relates to an optical probe for the simultaneous measurement of transmission radiation and scattered light radiation in liquid hydrocarbons which are transparent to the radiation, a sensor for the continuous measurement of emulsified water in liquid hydrocarbons, and to a measurement method for the qualitative or quantitative determination of water in liquid hydrocarbons.

It is known that contamination with water due to contact primarily with humid air, but also with storage containers and transport lines, takes place during the production, storage and transport of liquid fuels based on hydrocarbons. When the solubility limit is exceeded, emulsions of very finely divided water droplets initially form in the fuel base. Over time, the droplets become larger and then settle as a continuous sump phase on the bottom. Free water is therefore always present as an emulsified and/or continuous heavy phase in the fuels. It is also necessary for technical reasons (damage to engines) and safety reasons (air travel) to remove the sump from fuel containers or settling loops/bags of line systems by means of regular cleaning. During transport, for example, suspended water can be removed by means of coalescence separators or water absorbers in pipelines.

It has recently become known that additives to fuels can detrimentally affect the segregation of water. Furthermore, human error can also lead to inadvertent filling of tank systems with mixtures of fuel and water, or even pure water. For the aviation industry, very particularly stringent safety requirements moreover apply for understandable reasons. Attempts are therefore made to monitor the entire delivery chain of the fuels such as petroleum, diesel and especially kerosene from the refinery until fuelling, while rapidly detecting the free water content (meaning undissolved water as a disperse phase) and the presence of water instead of fuel and therefore promptly warning of hazards and preventing them.

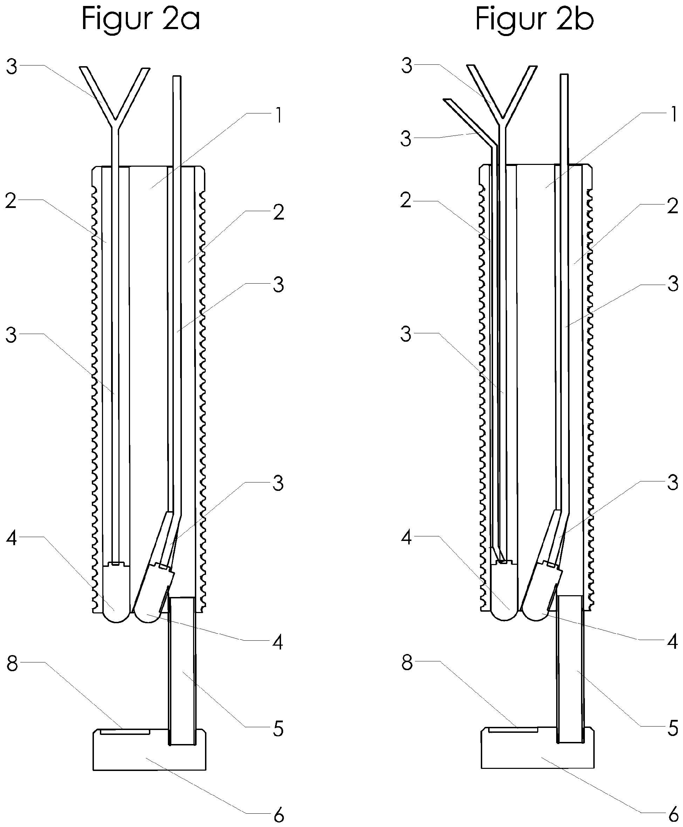

British Patent 1 460 623 describes an apparatus having a measurement chamber and a reference chamber for the determination of solid or liquid particles in liquids, for example water or soot particles in kerosene. A substantial component of the measurement device is an integrated dispersion unit for emulsified liquids. The measurement sample is divided into two streams before being introduced into the measurement chambers, the stream flowing to the reference chamber being heated in order to dissolve liquids. Suspended and/or dispersed particles can then be quantitatively determined by means of scattered light in the measurement chambers.
British Patent 1 554 309 describes a measurement device having a measurement chamber, an integrated dispersion unit, radiation source and detector for measuring the light absorption, and an alarm device for closing valves. Measuring from 40 to 50 cm, the measurement chamber has been felt to be too long and impracticable. It is only possible for emulsified (i.e. free) water in the fuel to be qualitatively determined by the measurement method. US patent 4,497,577 discloses a steam wetness measuring apparatus. Such steam wetness is measured as a relation between the intensity of a light beam and the intensity of a transmitted light beam or a scattered light beam. To measure such relationship, US 4,497,577 discloses for example in figures 2 or 7 probes which comprise one light beam emitter and a plurality of a light beam sensors. Such probe is meant to measure the wetness of the steam or the particle diameter of droplets within the steam. JP 2000 266668 discloses a sensor for monitoring a solution connected to a transmitted or absorbance measuring instrument. Such a sensor comprises only one light beam emitter as well as only one light beam sensor such that the light beam is travelling over a gap between the emitter and the sensor. A simultaneous measurement of scattered light and light absorption is not possible with only one sensor according to JP 2000 266668. US 5,007,740 discloses an optical probe for fluid light transmission properties. Such optical probe is constructed together and within a fluid sample chamber. Such an optical probe of US 5,007,740 is neither constructed too, nor possibly used for simultaneous measurement of liquid fluids of scattered light and light absorption according to the present application. No detector is yet known with which it is possible to determine free water continuously and quantitatively as a disperse phase in fuels and at the same time the presence of pure water in containers and lines. Quantitative determination should intended register quantities of from >0 to 50 mg, for example 1 to 30 mg of free water per kilogram of fuel, and serves for quality control and the prevention of excessively fast sump formation. The determination of the presence of pure water in the form of sump, bubbles, pools, continuous phases from the bottom up to the entire cross section of a line (i.e. the discrimination between fuel and water) in this case serves primarily for the nowadays very stringently set safety requirements.
It has been found that the aforementioned objects can be achieved in that only one beam path is used for measuring scattered light and light absorption and the wavelength of the radiation lies in the range of from 800 to 3000 nm. It has furthermore been found that such detectors can even be designed as immersion probes in which a defined measurement volume is provided, through which fuel constantly flows and which allows continuous measurements. The detectors can readily be configured in an explosion-protected embodiment.
The invention firstly relates to a probe for the simultaneous measurement of scattered light and light absorption at one wavelength or at two different wavelengths, comprising
a) optical elements for guiding and collimating transmission light, scattered light or radiation,
b) a first housing (1) having a first through-recess (2) and a second through-recess (2), the first and second through-recesses (2) connecting a first end of the first housing (1) and a second end of the first housing (1), the optical elements for guiding and collimating the transmission light or scattered light being comprised in the first and second through-recesses (2), the first housing (1) further having a first window (4) at a first end of the first through-recess (2) and a second window (4) at one end of the second through-recess (2), for the passage of transmission light, or light, and scattered light, the windows (4) being located at the first end of the first housing (1);
c) a second housing (6),
   - the second housing (6) being closed at a first end by a lid (7), the second housing (6) having a third recess (2) comprising the optical elements for guiding and collimating radiation and having a third window (4) for the passage of radiation at a second end of the second Housing (6), which is arranged opposite the first and second windows (4),
      or
   - the second housing (6) having no recess and having a mirror (8) arranged at a second end of the second housing (6) opposite the first and second windows (4);
d) at least one spacer (5) between the first end of the first housing (1) having the first and second windows (4) and the second end of the second housing having the third window (4), or the second end of the second housing having a mirror (8), which has a length of at least 3 mm;
e) a closure or a device for closure for connecting the first housing (1) to a container or a tube, by which the interior of the container or the tube is hermetically sealed from the surroundings, the closure being fitted to an outer surface of the first housing (1) said outer surface of the first housing (1) being designed as a screw thread.
The probe lies in direct contact with the fuel. The material for the housing and spacer is therefore selected so that it does not tumefy or become destroyed, for example corroded. Besides plastics such as e.g. polytetrafluoroethylene and other inert thermoplastics, it is possible to use ceramics, glass or quartz. Preferred materials are metals and metal alloys, for example aluminum, stainless steel and brass. Thermoplastics are particularly preferred since the housing can be produced economically by means of shaping methods such as e.g. injection moulding, pressing or extrusion methods.
The windows are transparent to the selected radiation and are fitted at the end of the recesses in a sealed fashion so that no fuel can enter the probe. Examples of materials are glasses, quartz, silicates, sapphire, metal oxides, semimetal oxides and plastics. The windows may be designed as lenses for the collimation of light or to generate coherent radiation. The diameter of the windows may, for example, be from 3 mm to 3 cm and preferably from 5 mm to 2 cm.

Gaskets or adhesives are preferably use for the sealing, and the window and gasket may be fastened for example by means of clamp rings or screw closures.

These recesses in the first housing are configured, for example as tubes, so that the diameter can receive optical elements for guiding and collimating both scattered light and transmission light. Examples of optical elements are lenses, prisms, optical filters, optical fibres and mirrors. A first recess (2) is provided in the first housing, in which light can be conducted through a hollow spacer to the second housing and scattered light can be received and conducted. When employing a transflection method, this recess may be used to receive optical elements for conducting only the scattered light to the measuring probe, since light can then be conducted through the second recess (2) onto an oppositely lying mirror (8) which replaces the third window (4). In this embodiment, the second housing fulfils the function of a frame for a mirror. The light is in this case it guided essentially perpendicularly onto the mirror surface, in which case transflection light can then also be measured via this second recess (2). Minor deviations from normal light incidence are nevertheless possible.

The recesses may be boreholes in the housing block.

The absorption of light is determined in a linear arrangement, whereas scattered light is normally detected at a defined angle. For guiding and collimating scattered light, it is therefore necessary to configure the first recess (2) in the first housing so that light and scattered light can be guided simultaneously in the first recess (2). The second recess (2) is preferably arranged on the opposite side from the first recess (2). This first recess may therefore also be partially used for conducting light to the third window (4) in the housing (6). The light conduction to the window (4) in the second housing (6) and for the measurement of absorption and scattering may take place by means of lenses and deviating elements. It has nevertheless been found highly advantageous to employ optical fibres or light waveguides for the light conduction, which are arranged separately in the recesses. Lenses are preferably fitted between the windows and light waveguides, in order to focus light onto the end of a light waveguide when the windows themselves are not designed as lenses. Frames for the light waveguides may be provided in the recesses in order to fix them, particularly in the region of the windows.

The scattered light is advantageously measured in the forward direction at an angle of < 90° to the radiation direction, preferably from 2 to 60°, more preferably from 4 to 40° and particularly preferably from 5 to 25°. The window (4) is arranged correspondingly to this angle and integrated into the first recess (2). In principle, it is also possible to measure back-scattering. In this case it is necessary to provide an additional window and optical elements in the second housing (6) in order to carry out the light conduction through the spacer and an additional recess in the second housing.

The geometrical shape of the probe is arbitrary per se; the housing and the spacer may, for example, be designed flatly as cuboids or preferably roundly as cylinders. The length of the first housing may for example be from 4 to 100, preferably from 6 to 80 and particularly preferably from 8 to 50 cm. When employed as an immersion probe, the overall length is dictated essentially by the diameter of the pipelines. The diameter of the first housing may for example be from 2 to 30, preferably from 4 to 20 and particularly preferably from 5 to 15 cm. The diameter and geometrical shape of the first and second housings are preferably designed equally. The shape of the bottom (7) of the second housing is expediently adapted to the shape of the containers and lines, in order to localize the measurement volume formed by the spacer as close as possible to the bottoms during operation.

The length of the second housing is generally less than that of the first housing and may, for example, be from 5 to 35% and preferably from 10 to 30% of the length of the first housing. The length is in particular substantially less when mirrors (8) are fitted on the second housing instead of a third window (4) for a transflection method. The second housing may then be very flatly configured and have a thickness of from 2 mm to 1 cm. In this variant according to the invention, the second housing may simply be configured as a mirror which is fastened on the spacer so that reflected light for measuring the absorption is conducted to the first window (4).

A recess for guiding light, optionally by means of light waveguides or optical fibres, is expediently arranged below a hollow spacer in the second housing, guided further along the wall to the bottom and along the bottom of the second housing, before being deviated at the wall to the third window (4) in the housing (6) in the direction of the first housing. The recesses along the walls may be obtained in a straightforward way by boring. A recess extending along the bottom may be obtained by boring or milling a cavity, which can be hermetically closed by a lid (screwing with a gasket).

When the radiation is intended to be reflected by a mirror (8) of the second housing, then the spacer may be designed as a solid rod and the second housing may be designed without recesses. A mirror may have approximately the same diameter as a window. In particular, a mirror for generating collimated radiation and/or for guiding the light may be designed as a concave mirror. In a simple embodiment, a mirror is mounted on a material block and connected to the first housing via a frame (5). It is also possible to integrate a mirror in the material block by polishing. A mirror may furthermore be firmly connected directly to the frame (5), which constitutes a particularly simple embodiment.

The spacer defines the optical layer thickness for measuring the absorption and generating scattered light. It preferably has a length of from 3 mm to 30 cm, preferably from 5 mm to 20 cm, more preferably from 5 mm to 15 cm and particularly preferably from 1 to 10 cm. The thickness of the spacer is selected so as to ensure sufficient mechanical stability (for example in relation to a flowing fuel). When light is intended to be conducted in the spacer, its thickness advantageously corresponds approximately to the diameter of the recess (2). When no mirrors are arranged in the second housing (6), the spacer is expediently connected to the first housing in extension of the first recess (2).

The parts of the probe according to the invention are configured and connected to one another so as to prevent fuel from entering. The housing and spacer may, for example, be made in one piece. The spacer may also be connected together with the two housings in a firm and sealed fashion by means of screwing, adhesive bonding, welding or soldering.

The closure is fitted on the outer wall at the head end of the first housing, for example to form an immersion probe. It may, for example, comprise screw, interlock or flange closures with which hermetic sealing can be achieved. To allow a sealed connection, pipelines and containers have corresponding inlets or glands as mating pieces. In order to achieve hermetic sealing, it is expedient to use gaskets. In a preferred embodiment, the surface of the first housing is designed as a screw thread, since simple and hermetically tight connections can thereby be achieved with fastening systems such as inlets in ball valves or union nuts.

The closure may be an integrated component of the first housing (1) having a circumferential recess, with or without a screw thread, so that support and firm connection is possible by placement or screwing (by means of a union nut) onto a gland of a pipeline or a container.

In a preferred embodiment a circumferential ring (10) for spacing control, which constitutes a part of a closure, is provided as an integrated component of the housing (1) on the upper end of the first housing (1) having a screw thread. This ring is used for support on a gland of a tube or container (12), optionally via a gasket (11) e.g. an O-ring. The closure may then be carried out by means of pressure via a flange (9), a union nut (9), by means of screwing or via a screw thread of the housing. Both the gland of the tube or container and the union nut may be provided with screw threads in order to connect the probe according to the invention in a firm and sealed fashion together with the integrated screw thread.

The openings of the first and second recesses for light conduction in the first housing open at the upper end of the housing (1). The recesses are designed to be open so that light waveguides can be guided through them. The ends may nevertheless also be closed with windows which, in particular, are designed as the lenses in order to focus light onto detectors. As an alternative, lenses may be arranged before and/or after the windows.

The determination of free water by means are scattered light is sensitive. With probes according to the invention, however, this sensitivity can be increased even further when a simultaneous measurement of scattered light is carried out at two opposite angles. To this end a recess for the light conduction may lie more centrally to the housing (1), so that a further window with a recess to receive scattered light and optical elements can be arranged in the first housing.

The sensitivity of the scattered light measurement can moreover be increased by detecting scattered light at a different wavelength from the light absorption. To this end a second light source advantageously with a higher power is used, for example high-power diodes. The light conduction may then take place via a glass fibre (3) or a glass fibre bundle (3). The second wavelength is expediently selected in the range of from 800 to 920 nm, preferably from 840 to 880 nm. High-power diodes with an emission wavelength of 860 nm are commercially available.

The probe according to the invention is suitable in particular as an immersion probe to be installed in pipelines and tanks for transporting and storing liquid fuels from oil refineries. These immersion probes ensure reliable and continuous determination of even very small amounts of free water in fuels to control the quality and for safety. With the absorption measurement, the presence of pure water can likewise be detected particularly rapidly with high reliability, even as pools on the bottoms of pipelines and tanks, which additionally increases the safety. The probe is therefore particularly suitable for a sensor with which a continuous measurement of the free water and the presence of water are automatically monitored. This sensor may be used as a signal transmitter for control elements to stop a fuel flow or determine the prompt removal of water sumps or bubbles.

The invention also relates to a sensor, comprising a probe according to the invention the sensor containing
a) one light source or two light sources for radiation in the range of from 800 to 3000 nm;
b) photodetectors for measuring the absorption and the scattered light;
c) a circuit board having elements for data processing and a program for the storage and comparison of measurement data in order to determine critical characteristic quantities, and elements for forwarding the data in the form of electrical signals; and optionally a signal transmitter for triggering an optical or acoustic warning signal.

The housing of the sensor part is fitted at the head end of the first housing of the probe and is firmly connected to the probe. Since electrical currents which represent a safety risk flow in the sensor part, the housing of the sensor part is particularly preferably configured in an explosion-protected embodiment. In an advantageous embodiment, all elements of the sensor part are arranged on a circuit board.

The sensor part may also be arranged separately from the probe and joined to the probe with flexible cables for conducting light. To this end cables, which are connected to the light sources and photodetectors of the sensor part, may be connected to the openings of the first and second recesses (2) for light conduction at the upper end of the housing (1).

The light source may comprise lamps, lasers or light-emitting diodes. In the case of lamps, interference filters must also be used. Lamps are not so advantageous owing to their size and the heat which they develop. Suitable lasers are semiconductor lasers and diode lasers. Light-emitting diodes, which allow a compact design owing to their small size, are particularly preferred. Preferred light sources for determining the absorption are those which emit light in the wavelength range of from 950 to 2200 nm. Light sources which emit in this range at the wavelength of the absorption bands of water, for example 970, 1155, 1470 or 1950 nm, are particularly preferred. If a second light source is used for determining the scattered light, then the wavelength will be selected to be shorter, for example in the range of from 800 to 920 nm, preferably from 840 to 880 nm. A second light source is advantageously associated with the first light source so that the light of both light sources is coupled into an optical conductor (glass fibre or glass fibre bundle) and guided to the measurement region.

Photodetectors are known and commercially available. They may comprise phototransistors or alternatively photodiodes. Photodetectors based on silicon are particularly suitable for the selected wavelength range. Detectors based on other semimetals and metals such as e.g. Ge, In, Ga, As, Sb, S and Se may nevertheless also be used.

Suitable signal amplifiers are arranged downstream of the photodetectors. They may for example comprise high-frequency alternating light amplifiers with an analogue or digital signal output.

A signal transmitter receives data in the form of electrical signals and emits a signal as an electrical pulse when a stored critical value is exceeded. With the pulse, an optical and/or acoustic warning signal may then be triggered or an electrical valve may be actuated in order to close a line.

Elements for data processing and a program for the storage and comparison of measurement data to determine critical quantities, and elements for forwarding data in the form of the electrical signals are known per se and familiar to the person skilled in the art, so that a description is unnecessary.

The invention also relates to a method for the quantitative, direct and continuous determination of free water as a disperse phase in liquid fuels, and the presence of pure water in tanks or pipelines for such fuels, which is characterized in that a sensor according to the invention is immersed in a tank or a pipeline and airtightly connected, the absorption and the scattered light are measured and converted into electrical signals, and the signals obtained are recorded.

For the quantitative determination, the sensor is calibrated beforehand with known quantities of free water in the fuel and then measurement values are compared with the calibrated values. With the method according to the invention, it is possible to measure quantities down to 1 mg or less of free water per kg of fuel, and the presence of water can be determined very rapidly. The method can therefore be used for quality control and also as an alarm system, in order to effectively prevent the influx of water or to remove accumulated water promptly.

The invention furthermore relates to a method for avoiding the filling of fuel tanks with too much or only with water, which is characterized in that a sensor according to the invention is immersed in a tank or a pipeline and airtightly connected, the absorption and the scattered light are measured and converted into electrical signals, and the signals obtained are recorded optionally as a log, the signal strength is compared with a critical reference signal and, if the signal strength exceeds the reference signal, an electrical pulse is triggered which leads to the triggering of an optical or acoustic warning signal and/or to a fuel flow being stopped.

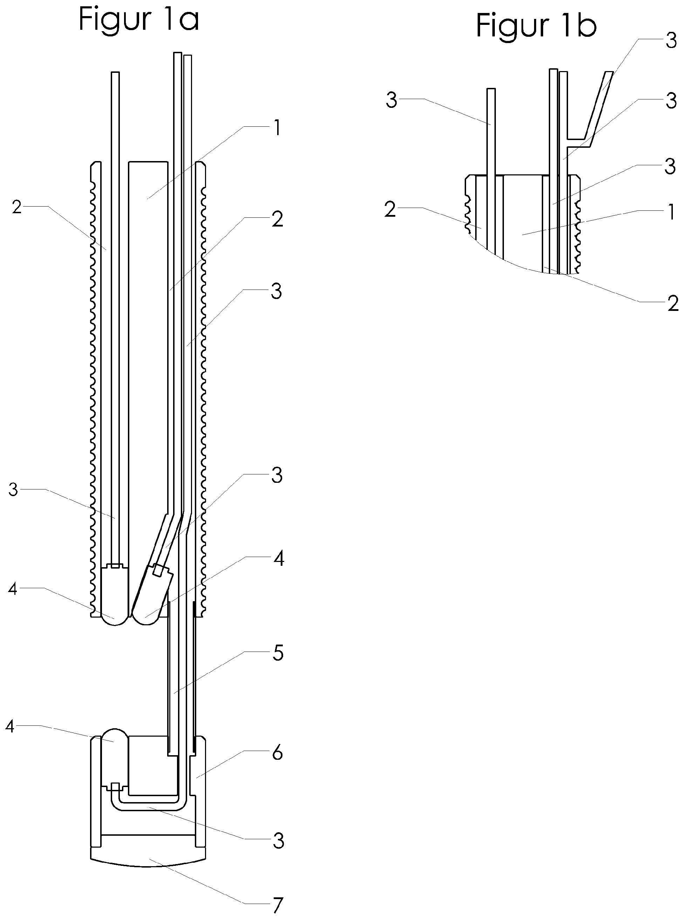

Figures 1 to 3 describe the invention in more detail.
Figure 1 a represents a cross section of a probe according to the invention, the wall of which is provided with a screw thread. A first window (4) and a second window (4), which open in a first recess (2) and a second recess (2) and are designed as lenses, are arranged in a first housing (1). The first recess (2) may be used as a channel for light guiding and for receiving scattered light. Optical fibres may be fastened as light waveguides (3) in the channels. The hollow space (5) connects the housing (1) to the second housing (6), which has a lid (7) and comprises a third window (4) for light to emerge to the second window (4).
Figure 1b shows an excerpt of a probe according to the invention with a bifurcated light waveguide (3) for shining light in from two different light sources.
Figure 2a represents a cross section of a probe according to the invention for transflection measurement, the wall of which is provided with a screw thread. A first window (4) and a second window (4), which open in a first recess (2) and a second recess (2) and are designed as lenses, are arranged in a first housing (1). The first recess (2) may be used as a channel for receiving and conducting scattered light via a light waveguide (3). The second recess (2) can be used for shining light in and conducting reflection light by a light waveguide (3). The space (5) connects the housing (1) to the second housing (6), which has a mirror (8) for generating transmission light.
Figure 2b shows a probe according to the invention for transflection measurement, according to Figure 2a but with an additional light waveguide (3) in the second recess (2) for coupling light in from two different light sources.

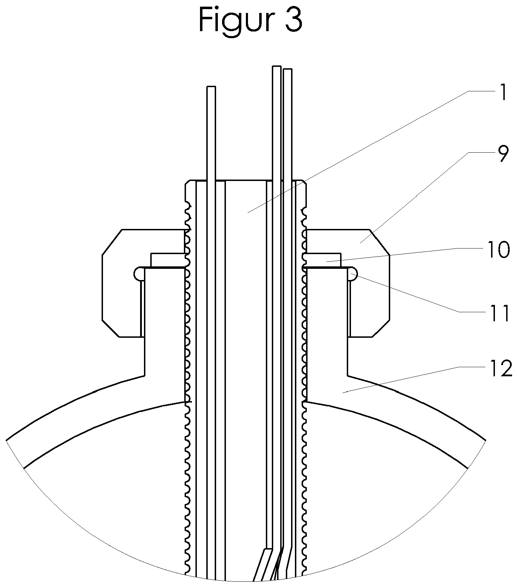

Figure 3 represents a cross section of a possible way of fastening the probe according to the invention, which is provided with a screw thread. At the upper end of the first housing (1) having a screw thread, there is a circumferential ring (10) as an integrated component of the housing (1). This ring is used for support on a gland of a tube or container (12). A union nut (9) connects the probe and tube or container (12) via a gasket (11), for example an O-ring.

## Claims

1. Probe for the simultaneous measurement of scattered light and light absorption at one wavelength or at two different wavelengths, comprising:
a) optical elements for guiding and collimating transmission light, scattered light or radiation;
b) a first housing (1) having a first through-recess (2) and a second through-recess (2), the first and second through-recesses (2) connecting a first end of the first housing (1) and a second end of the first housing (1), the optical elements for guiding and collimating the transmission light or scattered light being comprised in the first and second through-recesses (2), the first housing (1) further having a first window (4) at one end of the first through-recess (2) and a second window (4) at one end of the second through-recess (2), for the passage of transmission light, or light, and scattered light, the windows (4) being located at the first end of the first housing (1);
c) a second housing (6),
- the second housing (6) being closed at a first end by a lid (7), the second housing (6) having a third recess (2) comprising the optical elements for guiding and collimating radiation and having a third window (4) for the passage of radiation at a second end of the second housing (6), which is arranged opposite the first and second windows (4),
or
- the second housing (6) having no recess and having a mirror (8) arranged at a second end of the second housing (6) opposite the first and second windows (4);
d) at least one spacer (5) between the first end of the first housing (1) having the first and second windows (4) and the second end of the second housing (6) having the third window (4), or the second end of the second housing (6) having the mirror (8), which has a length of at least 3 mm;
e) a closure or a device for closure for connecting the first housing (1) to a container or a tube, by which the interior of the container or the tube is hermetically sealed from the surroundings, the closure being fitted to an outer surface of the first housing (1) said outer surface of the first housing (1) being designed as a screw thread.

2. Probe according to Claim 1, **characterized in that** the second window (4) is arranged opposite the window (4) in the housing (6).

3. Probe according to Claim 1, **characterized in that** the first window (4) is arranged to receive scattered light at an angle of < 90[deg.], with respect to the propagation direction of the light.

4. Probe according to Claim 1, **characterized in that** the spacer (5) has a length of from 3 mm to 30 cm.

5. Probe according to Claim 1, **characterized in that** the second housing (6) is designed as a mirror, or a mirror (8) is provided in the second housing.

6. Probe according to Claim 5, **characterized in that** the mirror (8) lies opposite the windows (4) of the first housing.

7. Sensor, which comprises an immersion probe according to one of the preceding claims 1-6, the sensor containing
a) one light source or two light sources for radiation in the range of from 800 to 3000 nm;
b) photodetectors for measuring the absorption and the scattered light;
c) a circuit board having elements for data processing and a program for the storage and comparison of measurement data in order to determine critical characteristic quantities, and elements for forwarding the data in the form of electrical signals; and optionally a signal transmitter for triggering an optical or acoustic warning signal.

8. Sensor according to Claim 7, **characterized in that** the second light source is associated with the first light source in such a way that the light of both light sources is coupled into an optical conductor (glass fibre or glass fibre bundle) and guided to the measurement region.

9. Sensor according to Claim 7, **characterized in that** both light sources comprise light-emitting diodes, the first light source emitting light with a wavelength of from 950 to 2200 nm with which the light absorption is measured, and the second light source emitting light with a wavelength of from 800 to 920 nm with which the scattered light is determined.

10. Method for the quantitative, direct and continuous determination of free water as a disperse phase in liquid fuels, and the presence of pure water in tanks or pipelines for such fuels, **characterized in that** a sensor according to Claim 8 is immersed in a tank or a pipeline and airtightly connected, the absorption and the scattered light are measured and converted into electrical signals, and the signals obtained are recorded.

11. Method for avoiding the filling of fuel tanks with too much or only with water, **characterized in that** a sensor according to Claim 8 is immersed in a tank or a pipeline and airtightly connected, the absorption and the scattered light are measured and converted into electrical signals, and the signals obtained are recorded optionally as a log, the signal strength is compared with a critical reference signal and, if the signal strength exceeds the reference signal, an electrical pulse is triggered which leads to the triggering of an optical or acoustic warning signal and/or to a fuel flow being stopped.

## Patentansprüche

1. Messsonde zur zeitgleichen Messung von gestreutem Licht und Lichtabsorption bei einer Wellenlänge oder bei zwei verschiedenen Wellenlängen, umfassend:
a) optische Elemente zur Führung und Einstellung von Transmissionslicht, gestreutem Licht oder Strahlung;
b) ein erstes Gehäuse (1), das eine erste durchgehende Ausnehmung (2) und eine zweite durchgehende Ausnehmung (2) hat, wobei die erste und die zweite durchgehende Ausnehmung (2) ein erstes Ende des ersten Gehäuses (1) und ein zweites Ende des ersten Gehäuses (1) verbindet, wobei die optischen Elemente zur Führung und Einstellung des Transmissionslichts oder des gestreuten Lichts in der ersten und der zweiten durchgehenden Ausnehmung (2) aufgenommen sind, wobei das erste Gehäuse (1) weiterhin ein erstes Fenster (4) an einem Ende der ersten durchgehenden Ausnehmung (2), und ein zweites Fenster (4) an einem Ende der zweiten durchgehenden Ausnehmung (2) hat für den Durchgang von Transmissionslicht, oder Licht, und gestreutem Licht, wobei die Fenster (4) an dem ersten Ende des Gehäuses (1) lokalisiert sind;
c) ein zweites Gehäuse (6),
- wobei das zweite Gehäuse (6) an einem ersten Ende durch einen Deckel (7) geschlossen ist, wobei das zweite Gehäuse (6) eine dritte Ausnehmung (2) hat, die die optischen Elemente zur Führung und Einstellung von Strahlung enthält, und ein drittes Fenster (4) hat für den Durchgang von Strahlung an einem zweiten Ende des zweiten Gehäuses (6), das gegenüber dem ersten und dem zweiten Fenster (4) angeordnet ist,
oder
- das zweite Gehäuse (6) keine Ausnehmung und einen Spiegel (8) hat, der an einem zweiten Ende des zweiten Gehäuses (6) gegenüber dem ersten und dem zweiten Fenster (4) angeordnet ist;
d) wenigstens einen Abstandhalter (5) zwischen dem ersten Ende des ersten Gehäuses (1), der das erste und das zweite Fenster (4) hat, und wobei das zweite Ende des zweiten Gehäuses (6) das dritte Fenster (4) hat, oder das zweite Ende des zweiten Gehäuses (6) den Spiegel (8) hat, der eine Länge von mindestens 3 mm hat;
e) einen Abschluss oder ein Mittel zum Abschluss zur Verbindung des ersten Gehäuses (1) mit einem Container oder einem Rohr, durch das das Innere des Containers oder Rohrs hermetisch von der Umgebung abgeschlossen wird, wobei der Abschluss auf eine äußere Fläche des ersten Gehäuses (1) angepasst wird, wobei die äußere Fläche des ersten Gehäuses (1) als ein Schraubgewinde ausgestaltet ist.

2. Messsonde nach Anspruch 1, **dadurch gekennzeichnet, dass** das zweite Fenster (4) gegenüber dem Fenster (4) in dem Gehäuse (6) angeordnet ist.

3. Messsonde nach Anspruch 1, **dadurch gekennzeichnet, dass** das erste Fenster (4) geeignet ist, gestreutes Licht unter einem Winkel von < 90[Grad] aufzunehmen, in Bezug auf die Propagationsrichtung des Lichts.

4. Messsonde nach Anspruch 1, **dadurch gekennzeichnet, dass** der Abstandhalter (5) eine Länge von 3 mm bis 30 cm hat.

5. Messsonde nach Anspruch 1, **dadurch gekennzeichnet, dass** das zweite Gehäuse (6) als ein Spiegel ausgebildet ist, oder ein Spiegel (8) in dem zweiten Gehäuse bereitgestellt ist.

6. Messsonde nach Anspruch 5, **dadurch gekennzeichnet, dass** der Spiegel (8) gegenüber den Fenstern (4) des ersten Gehäuses liegt.

7. Sensor, der eine Tauchsonde nach einem der vorangehenden Ansprüche 1- 6 aufweist, wobei der Sensor umfasst
a) eine Lichtquelle oder zwei Lichtquellen für Strahlung im Bereich von 800 bis 3000 nm;
b) Photodetektoren zur Messung der Absorption und des gestreuten Lichts;
c) eine Platine, die Elemente zur Verarbeitung von Daten und ein Programm zur Speicherung und zum Vergleich von Messdaten hat, um kritische charakteristische Größen zu bestimmen, und Elemente zur Weiterleitung der Daten in Form elektrischer Signale; und optional einen Signaltransmitter zur Auslösung eines optischen oder akustischen Warnsignals.

8. Sensor nach Anspruch 7, **dadurch gekennzeichnet, dass** die zweite Lichtquelle mit der ersten Lichtquelle derartig verbunden ist, dass das Licht von beiden Lichtquellen in einen optischen Leiter (Glasfaser oder Fiberglasbündel) gekoppelt und zur Messregion geführt ist.

9. Sensor nach Anspruch 7, **dadurch gekennzeichnet, dass** beide Lichtquellen lichtemittierende Dioden aufweisen, wobei die erste Lichtquelle Licht mit einer Wellenlänge von 950 bis 2200 nm emittiert, mit dem die Lichtabsorption gemessen wird, und die zweite Lichtquelle Licht mit einer Wellenlänge von 800 bis 920 nm emittiert, mit dem das gestreute Licht bestimmt wird.

10. Verfahren zur quantitativen, direkten und kontinuierlichen Bestimmung von freiem Wasser als Dispersionphase in flüssigen Treibstoffen, und der Anwesenheit von reinem Wasser in Tanks oder Pipelines für derartige Treibstoffe, **dadurch gekennzeichnet, dass** ein Sensor nach Anspruch 8 in einem Tank oder einer Pipeline eingetaucht und luftdicht verbunden ist, wobei die Absorption und das gestreute Licht gemessen werden und in elektrische Signale konvertiert werden, und die erhaltenen Signale aufgenommen werden.

11. Verfahren zur Vermeidung der Anfüllung von Treibstofftanks mit zu viel oder nur mit Wasser, **dadurch gekennzeichnet, dass** ein Sensor nach Anspruch 8 in einem Tank oder einer Pipeline eingetaucht und luftdicht verbunden ist, wobei die Absorption und das gestreute Licht gemessen werden und in elektrische Signale konvertiert werden, und die erhaltenen Signale optional als ein Protokoll aufgenommen werden, wobei die Signalstärke mit einem kritischen Referenzsignal verglichen wird und, falls die Signalstärke das Referenzsignal überschreitet, ein elektrischer Puls ausgelöst wird, der zur Auslösung eines optischen oder akustischen Warnsignals und/oder dazu führt, dass der Treibstofffluss gestoppt wird.

## Revendications

1. Sonde pour la mesure simultanée de lumière diffusée et d'absorption de lumière à une longueur d'onde ou à deux longueurs d'onde différentes, comprenant :
a) des éléments optiques pour guider et collimater une lumière de transmission, une lumière diffusée ou un rayonnement ;
b) un premier boîtier (1) présentant un premier évidement traversant (2) et un deuxième évidement traversant (2), les premier et deuxième évidements traversants (2) reliant une première extrémité du premier boîtier (1) et une seconde extrémité du premier boîtier (1), les éléments optiques pour guider et collimater la lumière de transmission ou la lumière diffusée étant compris dans les premier et deuxième évidements traversants (2), le premier boîtier (1) présentant en outre une première fenêtre (4) au niveau d'une extrémité du premier évidement traversant (2) et une deuxième fenêtre (4) au niveau d'une extrémité du deuxième évidement traversant (2), pour le passage de lumière de transmission, ou de lumière, et de lumière diffusée, les fenêtres (4) étant situées au niveau de la première extrémité du premier boîtier (1) ;
c) un second boîtier (6)
- le second boîtier (6) étant fermé au niveau d'une première extrémité par un couvercle (7), le second boîtier (6) présentant un troisième évidement (2) comprenant les éléments optiques pour guider et collimater un rayonnement et présentant une troisième fenêtre (4) pour le passage d'un rayonnement au niveau d'une seconde extrémité du second boîtier (6), qui est disposée en face des première et deuxième fenêtres (4),
ou
- le second boîtier (6) n'ayant pas d'évidement et présentant un miroir (8) disposé au niveau d'une seconde extrémité du second boîtier (6) en face des première et deuxième fenêtres (4) ;
d) au moins une entretoise (5) entre la première extrémité du premier boîtier (1) présentant les première et deuxième fenêtres (4) et la seconde extrémité du second boîtier (6) présentant la troisième fenêtre (4), ou la seconde extrémité du second boîtier (6) présentant le miroir (8), qui présente une longueur d'au moins 3 mm ;
e) une fermeture ou un dispositif de fermeture pour relier le premier boîtier (1) à un récipient ou un tube, par laquelle ou lequel l'intérieur du récipient ou du tube est hermétiquement isolé de l'environnement, la fermeture étant ajustée sur une surface extérieure du premier boîtier (1) ladite surface extérieure du premier boîtier (1) étant conçue comme un filetage.

2. Sonde selon la revendication 1, **caractérisée en ce que** la deuxième fenêtre (4) est disposée en face de la fenêtre (4) dans le boîtier (6).

3. Sonde selon la revendication 1, **caractérisée en ce que** la première fenêtre (4) est agencée pour recevoir de la lumière diffusée à un angle < 90 degrés, par rapport à la direction de propagation de la lumière.

4. Sonde selon la revendication 1, **caractérisée en ce que** l'entretoise (5) présente une longueur de 3 mm à 30 cm.

5. Sonde selon la revendication 1, **caractérisée en ce que** le second boîtier (6) est conçu comme un miroir, ou qu'un miroir (8) est prévu dans le second boîtier.

6. Sonde selon la revendication 5, **caractérisée en ce que** le miroir (8) se trouve en face de la fenêtre (4) du premier boîtier.

7. Capteur, qui comprend une sonde à immersion selon l'une des revendications précédentes 1 à 6, le capteur contenant
a) une source lumineuse ou deux sources lumineuses pour rayonnement dans la gamme de 800 à 3 000 nm ;
b) des photodétecteurs pour mesurer l'absorption et la lumière diffusée ;
c) une carte de circuit imprimé ayant des éléments pour le traitement de données et un programme pour le stockage et la comparaison de données de mesure dans le but de déterminer des quantités caractéristiques critiques, et des éléments pour faire suivre les données sous la forme de signaux électriques ; et optionnellement un émetteur de signal pour déclencher un signal d'alerte optique ou acoustique.

8. Capteur selon la revendication 7, **caractérisé en ce que** la seconde source lumineuse est associée à la première source lumineuse de telle sorte que la lumière des deux sources lumineuses est couplée dans un conducteur optique (fibre de verre ou faisceau de fibres de verre) et guidée vers la région de mesure.

9. Capteur selon la revendication 7, **caractérisé en ce que** les deux sources lumineuses comprennent des diodes électroluminescentes, la première source lumineuse émettant de la lumière avec une longueur d'onde de 950 à 2 200 nm avec laquelle l'absorption de lumière est mesurée, et la seconde source lumineuse émettant de la lumière avec une longueur d'onde de 800 à 920 nm avec laquelle la lumière diffusée est déterminée.

10. Procédé de détermination quantitative, directe et continue d'eau libre en tant que phase dispersée dans des carburants liquides, et de la présence d'eau pure dans des réservoirs ou des pipelines pour de tels carburants, **caractérisé en ce qu'**un capteur selon la revendication 8 est immergé dans un réservoir ou un pipeline et connecté hermétiquement, l'absorption et la lumière diffusée sont mesurées et converties en signaux électriques, et les signaux obtenus sont enregistrés.

11. Procédé pour éviter le remplissage de réservoirs de carburant avec trop d'eau ou seulement avec de l'eau, **caractérisé en ce qu'**un capteur selon la revendication 8 est immergé dans un réservoir ou un pipeline et connecté hermétiquement, l'absorption et la lumière diffusée sont mesurées et converties en signaux électriques, et les signaux obtenus sont enregistrés optionnellement en tant que journal, l'intensité du signal est comparée à un signal de référence critique et, si l'intensité du signal dépasse le signal de référence, une impulsion électrique est déclenchée ce qui conduit au déclenchement d'un signal d'alerte optique ou acoustique et/ou à l'arrêt d'un flux de carburant.
